(19) Europäisches Patentamt
European Patent Office
Office européen des brevets

(11) Veröffentlichungsnummer : **0 150 380**
**B1**

(12) **EUROPÄISCHE PATENTSCHRIFT**

(45) Veröffentlichungstag der Patentschrift : **07.09.88**

(51) Int. Cl.⁴ : **C 07 D233/58**, C 07 D235/06, C 07 D231/12, C 07 D249/08

(21) Anmeldenummer : **84115217.6**

(22) Anmeldetag : **12.12.84**

# (54) Verfahren zur Herstellung von N-vinylierten ungesättigten fünfgliedrigen N-Heterocyclen.

(30) Priorität : **24.12.83 DE 3347072**

(43) Veröffentlichungstag der Anmeldung : **07.08.85 Patentblatt 85/32**

(45) Bekanntmachung des Hinweises auf die Patenterteilung : **07.09.88 Patentblatt 88/36**

(84) Benannte Vertragsstaaten : **CH DE FR GB IT LI**

(56) Entgegenhaltungen :
**EP-A- 0 087 659**
**FR-A- 1 389 363**
**CHEMICAL ABSTRACTS, Band 69, Nr. 23, 2. Dezember 1968, Columbus, Ohio, USA I.I. GRANDBERG "Pyrazoles. LVII. Synthesis of 1- and 4-vinylpyrazoles" Seite 9 037, Spalte 1, Zusammenfassung-Nr. 96 564K**
**CHEMICAL ABSTRACTS, Band 94, Nr. 7, 16. Februar 1981, Columbus, Ohio, USA G.A. GAREEV "Synthesis of vinyl ethers of triazoles and tetrazole" Seite 571, Spalte 2, Zusammenfassung-Nr. 47 229n**
**CHEMICAL ABSTRACTS, Band 90, Nr. 5, 29. Jänner 1979, Columbus, Ohio, USA V.V. KALMYKOW "Synthesis of N-vinylimidazoles and their benzimidazoles", Seite 481, Spalte 2, Zusammenfassung-Nr. 38 840v**
**CHEMICAL ABSTRACTS, Band 83, Nr. 13, 29. September 1975, Columbus, Ohio, USA V.A. IVANOV, "Vinylation of imidazoles" Seite 547, Spalte 2, Zusammenfassung-Nr. 114 294t**

(73) Patentinhaber : **BAYER AG**
**Konzernverwaltung RP Patentabteilung**
**D-5090 Leverkusen 1 Bayerwerk (DE)**

(72) Erfinder : **Mues, Peter, Dr.**
**Breslauer Strasse 31**
**D 4150 Krefeld (DE)**
Erfinder : **Buysch, Hans-Josef, Dr.**
**Brandenburger Strasse 28**
**D 4150 Krefeld (DE)**

**EP 0 150 380 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

CHEMICAL ABSTRACTS, Band 79, Nr. 7, 20. August 1973, Columbus, Ohio, USA G.G. SKVORTSOVA "Behavior of nitrogen-containing five-membered heterocycles during vinylation" Seite 353, Spalte 1, Zusammenfassung-Nr. 42 414z

CHEMICAL ABSTRACTS, Band 62, Nr. 9, 26. april 1965, Columbus, Ohio, USA JAPAN GAS-CHEMICAL CO. "N-Vinylimidazole derivatives" Spalte 10 442, Zusammenfassung-Nr. 10 442f

Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung von N-vinylierten ungesättigten fünfgliedrigen N-Heterocyclen.

Zur Herstellung von N-vinylierten ungesättigten fünfgliedrigen N-Heterocyclen sind mehrere Verfahren bekannt : Umsetzung der N-H-Heterocyclen mit Acetylen unter Druck bei erhöhter Temperatur (Ann. 601, 81 (1956)), wobei umfangreiche Sicherheitsmaßnahmen und technisch aufwendige Apparaturen nötig sind ; Umsetzung der N-H-Heterocyclen mit Vinylacetat (Helv. Chim. Acta 43, 135 (1960)), wobei mit giftigen Quecksilbersalzen katalysiert werden muß ; Dehydratisierung von N-β-Hydroxyethyl-N-Heterocyclen an basischen Verbindungen (CA 69, 96, 564 k (1968)), wobei die Reaktion nicht generell anwendbar ist, da sie bislang nur von zwei Verbindungen bei hohen Reaktionstemperaturen (220 bzw. 300 °C) mit mäßigen Ausbeuten (50-65 %) eingegangen wird ; Dehydrohalogenierung von N-β-Chlorethyl-N-Heterocyclen mit Alkalimetallhydroxiden (FR 1 389 363), wobei die Chlorverbindung mit korrosivem und toxischem Thionylchlorid aus der entsprechenden Hydroxyverbindung hergestellt werden muß und Alkalimetallchloride zwangsweise anfallen sowie Umsetzung von N-H-Heterocyclen mit Vinylchlorid unter Druck bei erhöhter Temperatur [CA 62, 10 442 f (1965)], wobei umfangreiche sicherheitstechnische Maßnahmen erforderlich sind.

Aus der DE-OS 32 07 031 (entspricht EP 87 659) ist die Herstellung von cyclischen N-Vinylacylaminen durch Erhitzen von Kohlensäureestern cyclischer N-2-Hydroxyethylacylaminen in Gegenwart katalytischer Mengen alkalisch reagierender Verbindungen von Alkali- oder Erdalkalimetallen und anschließender Destillation des Reaktionsgemisches bekannt. Dieses Verfahren hat eine lediglich prinzipielle Analogie zum erfindungsgemäßen Verfahren.

Es wurde nun ein Verfahren zur Herstellung von N-vinylierten ungesättigten fünfgliedrigen N-Heterocyclen der Formel (I)

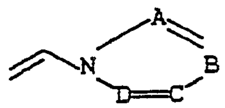

(I)

worin

A, B und C gleich oder verschieden sein können und für N, C-H, C-Hal, C-Alkyl oder C-Phenyl stehen können, mit der Maßgabe, daß A, B und C für maximal 2 weitere N-Atome im fünfgliedrigen Ring stehen,

D für C-H, C-Hal, C-Alkyl oder C-Phenyl stehen kann und

A und B oder C und D für den Fall, daß A, B und C nicht für N stehen, Bestandteil eines Benzolrings sein können, der gegebenenfalls durch Fluor, Chlor, Brom, eine Nitro-, Trihalogenmethyl-, Alkoxygruppe mit 1 bis 4 Kohlenstoffatomen, eine Carbalkoxygruppe mit 1 bis 4 Kohlenstoffatomen oder eine Dialkylaminogruppe mit 1 bis 4 Kohlenstoffatomen substituiert ist,

gefunden, das dadurch gekennzeichnet ist, daß man N-β-Hydroxyethyl-N-Heterocyclen der allgemeinen Formel (II)

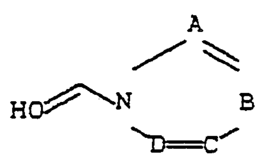

(II)

worin A, B, C und D die oben angegebene Bedeutung haben, mit Kohlensäureestern der Formel (III)

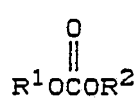

(III)

worin $R^1$ und $R^2$ gleich oder verschieden sind und für einen Alkylrest mit 1 bis 4 Kohlenstoffatomen stehen,

bei Temperaturen im Bereich von 100 bis 250 °C umsetzt, das entstandene Carbonat unter vermindertem Druck von 0,01 bis 300 mbar auf Temperaturen von 100 bis 270 °C erhitzt und anschließend das Reaktionsprodukt durch Destillation abtrennt.

Als Alkylrest mit 1 bis 4, bevorzugt 1 bis 2 Kohlenstoffatomen, der Formeln I, II und III seien genannt : der Methyl-, der Ethyl-, der n-Propyl-, der Isopropyl-, der n-Butyl-, der Isobutyl- und der tert.-Butylrest, bevorzugt der Methyl- und Ethylrest.

Als Alkoxygruppen mit 1 bis 4, bevorzugt 1 bis 2 Kohlenstoffatomen, der Formeln I und II seien genannt : der Methoxy-, der Ethoxy-, der n-Propyloxy-, der Isopropyloxy-, der n-Butyloxy-, der Isobutyloxy- und der tert.-Butyloxyrest, bevorzugt der Methoxy- und Ethoxyrest, und als Carbalkoxygruppen der

Formeln I und II mit 1 bis 4, bevorzugt 1 bis 2 Kohlenstoffatomen seien genannt: Methoxycarbonyl, Ethoxycarbonyl, n-Propyloxycarbonyl, Isopropyloxycarbonyl, n-Butyloxycarbonyl, Isobutyloxycarbonyl und tert.-Butyloxycarbonyl, bevorzugt Methoxycarbonyl und Ethoxycarbonyl.

Als Halogene seien genannt: Fluor, Chlor und Brom, bevorzugt Fluor und Chlor.

Zum Beispiel können als Verbindungen der Formel (II) in das erfindungsgemäße Verfahren eingesetzt werden: 1-Ethanol-1H-imidazol, 1-Ethanol-2-methyl-1H-imidazol, 1-Ethanol-1H-benzimidazol, 1-Ethanol-2-methyl-1H-benzimidazol, 4,5-Diphenyl-1-ethanol-1H-imidazol, 1-Ethanol-1H-pyrazol, 4-Chlor-1-ethanol-1H-pyrazol, 1-Ethanol-1H-1,2,4-triazol, 1-Ethanol-1H-benzotriazol, 1-Ethanol-5-nitro-1H-benzotriazol.

Die o. g. N-β-Hydroxyethyl-N-heterocyclen können auf einfache Weise durch Umsetzung von Glykolcarbonat mit den entsprechenden N-H-Heterocyclen in Substanz oder durch Addition von Ethylenoxid unter hierfür üblichen Reaktionsbedingungen (s. Houben-Weyl, « Methoden zur Herstellung und Umwandlung dreigliedriger, cyclischer Ether (1,2-Epoxide) », S. 367 ff, 4. Auflage, 1965) hergestellt werden.

Nach dem erfindungsgemäßen Verfahren wird die Umsetzung der N-β-Hydroxyethyl-N-heterocyclen der allgemeinen Formel II mit Kohlensäureestern der allgemeinen Formel III bei Temperaturen im Bereich von etwa 100 bis 250 °C, vorzugswzeise 110 bis 160 °C, durchgeführt.

Als Kohlensäureester der Formel III seien solche genannt, deren Alkylreste 1 bis 4 Kohlenstoffatome, bevorzugt 1 bis 2 Kohlenstoffatome enthalten, wie der Dimethyl-, Diethyl-, Di-n-propyl-, Diisopropyl-n Di-n-butyl-, Diisobutyl- und der Di-tert.-butylester der Kohlensäure, bevorzugt der Kohlensäuredimethyl- und der Kohlensäurediethylester.

Bezogen auf den eingesetzten N-β-Hydroxyethyl-N-heterocyclus der Formel (II) kann der Kohlensäureester im Unterschuß, im Überschuß oder in äquimolaren Mengen eingesetzt werden. Bevorzugt wird ein Molverhältnis der einzusetzenden β-Hydroxyethylverbindung zum einzusetzenden Kohlensäureester gewählt, das etwa 1 : 1 bis 1 : 10, besonders bevorzugt 1 : 2 bis 1 : 6 beträgt.

Die Umsetzung der β-Hydroxyverbindungen der allgemeinen Formel (II) mit dem Kohlensäuredialkylester kann mit oder ohne ein inertes Lösungsmittel durchgeführt werden. Als inerte organische Lösungsmittel kommen z. B. in Frage: Xylole oder Ether, wie Anisol.

Die Umsetzung der β-Hydroxyverbindungen der allgemeinen Formel (II) mit den Kohlensäuredialkylestern sowie die anschließende Spaltung können mit oder ohne Katalysatoren durchgeführt werden. Bevorzugt wird die Umsetzung und die Spaltung ohne Katalysator durchgeführt. Eine Zugabe von Katalysatoren, wie sie in der DE-OS 3 207 031 beschrieben werden, beeinflussen die erfindungsgemäße Umsetzung jedoch nicht nachteilig. In manchen Fällen kann die Verwendung von Katalysatoren auch vorteilhaft sein. Die in der DE-OS 3 207 031 beschriebenen Katalysatoren können in den dort angegebenen Mengenverhältnissen eingesetzt werden. Beispielsweise eignen sich die alkalisch reagierenden Verbindungen von Alkali- oder Erdalkalimetalen, wie die Hydroxide, Alkoholate oder die alkalisch reagierenden Salze, wie die Carbonate oder die Carboxylate des Lithiums, Natriums, Kaliums, Magnesiums, Calciums, Strontiums oder des Bariums. Bevorzugt werden die alkalisch reagierenden Verbindungen der Alkali- oder Erdalkalimetalle in Mengen von 0,001 bis 5 Gew.-% eingesetzt.

Die N-Vinylverbindungen der allgemeinen Formel (I) können leicht durch Erhitzen des nach erfolgter Umsetzung der N-β-Hydroxyethylverbindungen der allgemeinen Formel (II) mit Kohlensäuredialkylestern erhaltenen Reaktionsgemisches auf Temperaturen von etwa 100 bis 270 °C, bevorzugt 140 bis 230 °C, unter vermindertem Druck bei etwa 0,01 bis 300, bevorzugt 0,01 bis 100 mbar, erhalten werden. Dabei werden die Carbonate der entsprechenden N-β-Hydroxyethylverbindungen der allgemeinen Formel (II) gespalten.

Zur weiteren Reinigung des dabei erhaltenen Produktes kann unter vermindertem Druck bei etwa 0,01 bis 200, bevorzugt 0,01 bis 20 mbar, redestilliert werden.

Der bei der Spaltreaktion entstehende Alkohol, beispielsweise Methanol oder Ethanol, wird zweckmäßigerweise in einer mit Trockeneis beschickten Kühlfalle kondensiert, während das Kohlendioxyd entweder in einer mit flüssigem Stickstoff gekühlten Falle oder in einer geeigneten Flüssigkeit, wie Ethanolamin oder Ethylendiamin, unter Carbaminatbildung aufgefangen wird.

N-vinylierte ungesättigte fünfgliedrige N-Heterocyclen der allgemeinen Formel (I) sind wertvolle Vorprodukte zur Herstellung von Pflanzenschutzmitteln (DE-OS 2 554 790 und 2 558 163). Sie können polymerisiert werden und dienen u. a zur Herstellung von lichtempfindlichen Beschichtungen (DE-OS 2 327 732, DE-OS 2 509 019, US 3 820 993) und Ionenaustauschern (CA 94, 123 939 m (1981)).

## Beispiel 1

N-Vinylimidazol (1-Ethenyl-1H-imidazol)

224 g (2 Mol) 1-Ethanol-1H-imidazol, 944 g (8 Mol) Di-ethylcarbonat und 2 g Phenothiazin als Polymerisationsinhibitor werden an einer 1,2 m hohen Füllkörperkolonne während 1,5 h auf 120-130 °C Innentemperatur erhitzt. Über Kopf werden 92 g Ethanol (100 % d. Th.) und 43 g Diethylcarbonat abdestilliert, wobei gegen Ende der Umesterung das Ethanol durch stufenweise Verringerung des Druckes bis auf 300 mbar entfernt wird. Nach Abdestillieren des überschüssigen Diethylcarbonats unter 30 mbar erhält man 344 g Rückstand.

Dieser wird unter Eintropfen in einen vorgeheizten Kolben unter Rühren und vermindertem Druck bei 8 mbar und bei einer Innentemperatur von 180-190 °C gespalten. Es gehen 48 g 1-Ethanol-1H-imidazol (Ausgangsmaterial) und 111 g Vinylimidazol ($Kp._{4,1}$ 64 °C ; 75 % d. Th., bezogen auf umgesetztes 1-Ethanol-1H-imidazol) über.

Beispiel 2

N-Vinylbenzimidazol

164 g (1 Mol) N-β-Hydroxyethylbenzimidazol, 472 g (4 Mol) Diethylcarbonat und 1 g Phenothiazin als Polymerisationsinhibitor werden — wie in Beispiel 1 beschrieben — umgesetzt. Nach Entfernung des überschüssigen Diethylcarbonats verbleiben 233 g Rückstand, der unter Eintropfen in einen vorgeheizten Kolben unter Rühren und vermindertem Druck bei 3 mbar und einer Innentemperatur von 180-190 °C gespalten wird. Es gehen 20 g Ausgangsmaterial und 76 g N-Vinylbenzimidazol ($Kp._{0,05}$ 73-75 °C, 60 % d. Th., bezogen auf umgesetztes N-β-Hydroxyethylbenzimidazol) über.

Beispiel 3

1-Ethenyl-1H-1,2,4-triazol

79 g (0,7 Mol) 1-Ethanol-1H-1,2,4-triazol, 354 g (3 Mol) Diethylcarbonat, 0,7 g $K_2CO_3$ und 0,7 g Phenothiazin als Polymerisationsinhibitor werden — wie in Beispiel 1 beschrieben — umgesetzt. Nach Entfernung des überschüssigen Diethylcarbonats bei 50 mbar verbleiben 122 g Rückstand, der unter Eintropfen in einen vorgeheizten Kolben unter Rühren und vermindertem Druck bei 30-40 mbar und einer Temperatur von 170-180 °C gespalten wird. Es gehen 14 g Ausgangsmaterial und 30 g 1-Ethenyl-1H-1,2,4-triazol ($Kp._{18}$ 68-72 °C, 55 % d. Th., bezogen auf umgesetztes 1-Ethanol-1H-1,2,4-triazol) über.

Beispiel 4

N-Vinylbenztriazol

163 g (1 Mol) N-β-Hydroxyethylbenztraizol, 472 g (4 Mol) Diethylcarbonat und 1 g Phenothiazin als Polymerisationsinhibitor werden — wie in Beispiel 1 beschrieben — umgesetzt. Nach Entfernung des überschüssigen Diethylcarbonats verbleiben 211 g Rückstand, der unter Eintropfen in einen vorgeheizten Kolben unter Rühren und vermindertem Druck bei 10 mbar und einer Temperatur von 190-200 °C gespalten wird. Es gehen 21 g Ausgangsmaterial und 96 g N-Vinyltriazol ($Kp._{0,04}$ 63-65 °C, 76 % d. Th., bezogen auf umgesetztes N-β-Hydroxyethylbenztriazol) über.

Beispiel 5

N-Vinylpyrazol

100 g (0,9 Mol) N-β-Hydroxyethylpyrazol, 420 g (3,6 Mol) Diethylcarbonat, 0,9 g $K_2CO_3$ und 0,9 g Phenothiazin als Polymerisationsinhibitor werden — wie in Beispiel 1 beschrieben — umgesetzt. Nach Entfernung des überschüssigen Diethylcarbonats bei 50 mbar verbleiben 190 g Rückstand, der auf 170-190 °C unter Rühren und vermindertem Druck erst bei 90 mbar und gegen Ende der Reaktion bei 20 mbar erhitzt wird. Es gehen 36 g Ausgangsmaterial und 35 g N-Vinylpyrazol ($Kp._{50}$ 63 °C, 65 % d. Th., bezogen auf umgesetztes N-β-Hydroxy-ethylpyrazol) über.

**Patentansprüche**

1. Verfahren zur Herstellung von N-vinylierten ungesättigten fünfgliedrigen N-Heterocyclen der Formel

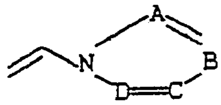

worin

A, B und C gleich oder verschieden sein können und für N, C-H, C-Hal, C-Alkyl oder C-Phenyl stehen können, mit der Maßgabe, daß A, B und C für maximal 2 weitere N-Atome im fünfgliedrigen Ring stehen,

D für C-H, C-Hal, C-Alkyl oder C-Phenyl stehen kann und

A und B oder C und D für den Fall, daß A, B und C nicht für N stehen, Bestandteil eines Benzolrings

sein können, der gegebenenfalls durch Fluor, Chlor, Brom, eine Nitro-, Trihalogenmethyl-, Alkoxygruppe mit 1 bis 4 Kohlenstoffatomen, eine Carbalkoxygruppe mit 1 bis 4 Kohlenstoffatomen oder eine Dialkylaminogruppe mit 1 bis 4 Kohlenstoffatomen substituiert ist,
dadurch gekennzeichnet, daß man N-β-Hydroxyethyl-N-Heterocyclen der allgemeinen Formel

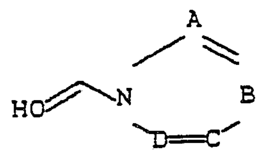

(II)

worin A, B, C und D die oben angegebene Bedeutung haben, mit Kohlensäureestern der Formel

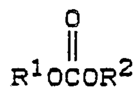

worin $R^1$ und $R^2$ gleich oder verschieden sind und für einen Alkylrest mit 1 bis 4 Kohlenstoffatomen stehen,
**bei Temperaturen im Bereich von 100 bis 250 °C umsetzt, das entstandene Carbonat unter vermindertem** Druck von 0,01 bis 300 mbar auf Temperaturen von 100 bis 270 °C erhitzt und anschließend das Reaktionsprodukt durch Destillation abtrennt.

2. Verfahren nach Anspruch 1, dadurch gekennzeichnet, daß man als Kohlensäureester Dimethyl-, Diethyl-, Di-n-propyl-, Diisopropyl-, Di-n-butyl-, Diisobutyl- und Di-tert.-butyl-Ester der Kohlensäure eingesetzt werden.

**Claims**

1. Process for the preparation of N-vinylated unsaturated five-membered N-hetrerocyclic compounds of the formula

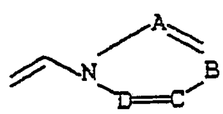

wherein
A, B and C can be identical or different and can represent N, C-H, C-Hal, C-alkyl or C-phenyl, with the proviso that A, B and C represent a maximum of 2 further N atoms in the five-membered ring, and
D can represent C-H, C-Hal, C-alkyl or C-phenyl, or
A and B, or C and D, where A, B and C do not represent N, can be the constituent of a benzene ring, which is optionally substituted by fluorine, chlorine, bromine, a nitro or trihalogenomethyl group, an alkoxy group with 1 to 4 carbon atoms, a carbalkoxy group with 1 to 4 carbon atoms or a dialkylamino group with 1 to 4 carbon atoms,
characterised in that N-β-hydroxyethyl-N-heterocyclic compounds of the general formula

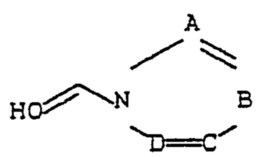

(II)

wherein A, B, C and D have the abovementioned meaning, are reacted with carbonic acid esters of the formula

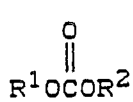

wherein $R^1$ and $R^2$ are identical or different and represent an alkyl radical with 1 to 4 carbon atoms, at temperatures in the range from 100 to 250 °C, the resulting carbonate is heated under a reduced pressure of 0.01 to 300 mbar to temperatures of 100 to 270 °C and the reaction product is then separated off by distillation.

2. Process according to Claim 1, characterised in that dimethyl, diethyl, di-n-propyl, diisopropyl, di-n-butyl, diisobutyl or di-tert.-butyl carbonate is used as the carbonic acid ester.

**Revendications**

1. Procédé de production de N-hétérocycles pentagonaux non saturés N-vinylés de formule

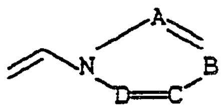

dans laquelle

A, B et C peuvent être identiques ou différents et peuvent rperésenter N, un groupe C-H, C-Hal, C-alkyle ou C-phényle, sous réserve que A, B et C représentent au maximum 2 autres atomes d'azote dans le noyau pentagonal,

D peut être un groupe C-H, C-Hal, C-alkyle ou C-phényle et

A et C ou C et D peuvent, au cas où A, B et C ne représentent pas de l'azote, être un constituant d'un noyau benzénique qui est substitué le cas échéant par du fluor, du chlore, du brome, un groupe nitro, trihalogénométhyle, alkoxy ayant 1 à 4 atomes de carbone, un groupe carbalkoxy ayant 1 à 4 atomes de carbone ou un groupe dialkylamino ayant 1 à 4 atomes de carbone,

qui est caractérisé en ce qu'on fait réagir à des températures comprises dans l'intervalle de 100 à 250 °C de N-β-hydroxyéthyl-N-hétérocycles de formule générale (II)

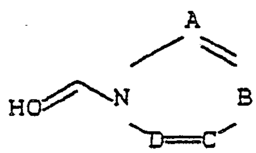

(II)

dans laquelle A, B, C et D ont la définition indiquée ci-dessus, avec des esters d'acide carbonique de formule

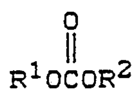

dans laquelle $R^1$ et $R^2$ sont identiques ou différents et représentent un reste alkyle ayant 1 à 4 atomes de carbone,

on chauffe le carbonate produit, sous pression réduite de 0,01 à 300 mbars, à des températures de 100 à 270 °C, puis on sépare par distillation le produit réactionnel.

2. Procédé suivant la revendication 1, caractérisé en ce qu'on utilise comme esters d'acide carbonique les esters de diméthyle, de diéthyle, de di-n-propyle, de disopropyle, de di-n-butyle, de diisobutyle et de di-tertio-butyle de l'acide carbonique.